# EUROPEAN PATENT APPLICATION

(11) **EP 4 800 435 A1**
(43) Date of publication of application: **02.09.2026**
(21) Application number: 25160717.2
(22) Date of filing: 27.02.2025
(51) Int. Cl.: G01S 13/06, G01S 13/46, G01S 13/86, G01S 17/06, G01S 17/46, G01S 17/86, H04B 10/114

(54) **METHOD OF DETERMINING A LOCATION OF A DEVICE IN A HEALTHCARE SETTING**

(71) Applicant: Baxter Medical Systems GmbH + Co. KG, 07318 Saalfeld (DE)
(72) Inventor: MAIER, Patrick, 07318 Saalfeld (DE); NTOVLIATIDOU, Maria, 07318 Saalfeld (DE); AMALANESAN, Antony L. F., 07318 Saalfeld (DE); SCHREITER, Luzie, 07318 Saalfeld (DE); LUKSCH, Tobias, 07318 Saalfeld (DE); NEUPERT, Carsten, 07318 Saalfeld (DE)
(74) Representative: Reddie & Grose LLP

(57) **Abstract**

A method of determining a location of a device in a healthcare setting. The method comprises: detecting electromagnetic radiation emitted or reflected by a device to be detected; determining at least one pattern of the detected electromagnetic radiation; and, in dependence on information encoded by the at least one pattern, determining at least one location characteristic of the device. An apparatus for determining a location of a device in a healthcare setting, and a system for determining a location of a device in a healthcare setting, are also provided.

## Description

### TECHNICAL FIELD

The present disclosure relates to a method of determining a location of a device in a healthcare setting. In particular, the disclosure relates to a method comprising determining at least one location characteristic of the device in dependence on information encoded by at least one pattern of detected electromagnetic radiation. The disclosure further relates to an apparatus, and to a system, for determining a location of a device in a healthcare setting.

### BACKGROUND

Precisely determining the location of objects in three-dimensional spaces is required in many applications. For example, precise locating of objects is required for augmented reality (AR), positioning, navigation, etc. in various sectors such as manufacturing, healthcare, etc. In healthcare settings, in particular in operating rooms, determination of a precise location of a device is required. For example, a device such as a C-arm, a display, or a surgical light may be provided in various positions, poses or configurations, and orientations.

Different types of sensors may be used to determine the location of objects. For example, it is known to use depth sensors, in particular so called time-of-flight (ToF) sensors, to create a three-dimensional representation of the space, e.g. an operating room, and the objects within the space, e.g. devices in an operating room. ToF sensors, also referred to as ToF cameras, measure a distance between the sensor and the target by determining a time-of-flight, or round trip time of light (electromagnetic radiation), from the sensor to the target and back from the target to the sensor.

The inventors have found that when trying to locate objects in a 3D space with depth sensors, occlusion of objects may be a problem. Objects are often not visible completely to the depth sensors because of other objects, or persons, in the line of sight of the sensors.

The inventors have appreciated the need for improved locating of devices, such as surgical lights, displays, carts, surgical tables, stretchers, hospital beds, surgical booms, and C-arms, in healthcare settings like operating rooms, and in particular, improved precision in determining the location of target devices that may be partly occluded.

### SUMMARY OF THE DISCLOSURE

The present disclosure provides a method of determining a location of a device, an apparatus for determining a location of a device, and a system for determining a location of a device, as defined in the appended claims, to which reference should now be made.

According to a first aspect of the present disclosure, there is provided a method of determining a location of a device in a healthcare setting. The method comprises: detecting electromagnetic radiation emitted or reflected by a device to be located; determining at least one pattern of the detected electromagnetic radiation; and, in dependence on information encoded by the at least one pattern, determining at least one location characteristic of the device.

Determining a location (position, configuration/pose, orientation) or geometry of an object in a three dimensional space may be challenging if a portion of the object is occluded. In a healthcare setting, such as an operating room, it is common for a device, the location of which may have to be determined precisely for positioning, navigation, or collision prevention, to be partly occluded by other devices or persons. The method of the first aspect, by using information encoded in a pattern of electromagnetic radiation emitted or reflected by a target device to determine at least one location characteristic of the device may allow for improved determination of a 3D geometry of the object, by helping to identify the visible portion of the object. In particular, the method may significantly improve determination of a 3D geometry of a device which is partially occluded.

By way of example, if the device is a C-arm, precise locating of the C-arm may allow for improved imaging, or a reduced need for additional imaging. In particular, surgical staff may move the C-arm into position, image at the desired position, and then move the arm away to progress surgery. Following the subsequent step(s) in the surgical procedure, a second, comparison, image may be required. However, if the comparison image is not take in exactly the same position as the first image, it may be less suitable for comparison, or the position may have to be adjusted, and a further image taken. This may lead to a patient being exposed to unnecessary additional radiation.

As used herein, a "location" may include a position of the device in a three-dimensional space, a configuration and/or pose of the device, and an orientation of the device. A "configuration" of the device may relate to the dimensions of the device, which may depend on the positioning of parts of the device relative to one another, or on optional components of the device which may be added to the device. As such, the configuration may include dimensions of the device. An orientation of the device may relate to an angle/rotation at which the device is arrange.

As used herein, determining a location of a device/locating a device refers to determining at least one of a position, a configuration/pose, and an orientation of the device.

As used herein, a "device to be located" refers to a device a location of which is to be determined. The "device to be located" may be referred to as a target device. The method may be referred to as a method of determining a location of a target device in a healthcare setting.

The method may be a method of determining a location of a medical device in a healthcare setting. Examples of medical devices include C-arms, surgical lights, surgical tables, and the like.

The method may further comprise providing, on the device to be located, at least one electromagnetic radiation modifier configured to cause a pattern of electromagnetic radiation to be emitted or reflected by the device. Advantageously, by providing at least one radiation modifier on the device, existing devices may be retrofitted for improved locating. Further, by providing at least one electromagnetic radiation modifier, specific information may be encoded by controlling the at least one pattern being caused.

Providing at least one electromagnetic radiation modifier may comprise at least one of: painting; adhering; and fastening.

Providing at least one electromagnetic radiation modifier may comprise retrofitting an existing device. In other examples, providing at least one electromagnetic radiation modifier may comprise applying the at least one electromagnetic radiation modifier during manufacture of the device.

Optionally, the at least one electromagnetic radiation modifier may be removable. Further optionally, the at least one electromagnetic radiation modifier may be reusable.

In some embodiments, providing at least one electromagnetic radiation modifier may comprise providing at least one electromagnetic radiation modifier on each side of the device. Advantageously, this may allow for at least one pattern caused by the at least one electromagnetic radiation modifier to be detectable, independently of the orientation of the device.

Optionally, a plurality of electromagnetic radiation modifiers are provided, each electromagnetic radiation modifier configured to cause a pattern of electromagnetic radiation to be emitted or reflected by the device. By providing a plurality of electromagnetic radiation modifiers, a plurality of patterns of electromagnetic radiation may be caused, thus improving versatility, in particular if a partially occluded device is to be located. If a plurality of electromagnetic radiation modifiers is provided, information being encoded in each pattern, only one pattern caused by one of the plurality of modifiers may need to be visible in the detected electromagnetic radiation to improve determination of the location of the device. As such, even a partially obscured device could be more precisely located, provided one pattern encoding information is determinable.

Further optionally, each pattern encodes different information. By encoding different information in at least two patterns, further improved locating may be achieved, because one can determine, if only one pattern is visible or unoccluded, which pattern is visible or unoccluded, and by using any further encoded information, improved accuracy may be achieved. If two or more patterns are visible or unoccluded, the information encoded in each pattern may be used to further enhance precision.

The, or each, electromagnetic radiation modifier may comprise at least one of: a passive electromagnetic radiation modifier and an active electromagnetic radiation source configured to emit electromagnetic radiation. Advantageously, a passive electromagnetic radiation modifier may be simple, because it does not require, e.g. electrical, activation. Advantageously, an active electromagnetic radiation source does not rely on external electromagnetic radiation, thus it may allow the method to be used independently of external factors.

In some examples, the, or each, passive electromagnetic radiation modifier comprises at least one of: a reflector configured to reflect a greater proportion of incident electromagnetic radiation than the device, and an absorber configured to absorb a greater proportion of incident electromagnetic radiation than the device. Advantageously, a reflector may allow for relatively low intensity radiation to be detected, as a greater proportion of incident electromagnetic radiation is reflected. On the other hand, an absorber may be suitable only if incident electromagnetic radiation is sufficiently high, so that the radiation reflected by the device is sufficiently high to be distinguished from the (smaller proportion) reflected by the absorber. However, an absorber may be particularly suitable if the device reflects a relatively greater proportion of incident electromagnetic radiation.

The, or each, reflector may preferably be a retroreflector. A retroreflector may facilitate determination of a pattern, as it reflects radiation with minimal scattering. As such, a retroreflector may provide a maximum contrast in an electromagnetic radiation image, particularly an infrared radiation image.

In some examples, the, or each, active electromagnetic radiation source is configured to emit electromagnetic radiation continuously, or intermittently. Advantageously, electromagnetic radiation being emitted continuously may facilitate determination of a pattern. On the other hand, intermittently emitting electromagnetic radiation may allow for information to be encoded in the intermittent signal. For example, the intermittent active electromagnetic radiation source may be configured to emit electromagnetic radiation in a specific sequence, encoding information in a temporal manner. In one example, the active radiation source may be configured to emit electromagnetic radiation in a Morse code.

The, or each, active electromagnetic radiation source may comprise at least one light-emitting diode (LED), in particular an infrared (IR) LED.

In some examples, the, or each, passive electromagnetic radiation modifier may comprise at least one geometric feature on a surface of the device, the geometric feature comprising at least one of: an indentation and a projection.

The, or each, electromagnetic radiation modifier may be arranged to form indicia in the pattern. By arranging the electromagnetic radiation modifier to form indicia in the pattern, information may be easily encoded in the pattern.

In some examples, the indicia comprise at least one machine-readable code, for example a barcode or a barcode-based code. Advantageously, a barcode, or a barcode-based code, may allow for a relatively large amount of information to be encoded in a simple manner.

Optionally, the, or each, barcode comprises a linear barcode. Advantageously, a linear barcode may be easy to read and may easily encode information. One suitable example is an Interleaved 2 of 5 (ITF) barcode. ITF is a relatively high density (i.e. space-efficient) numeric-only barcode. Advantageously, because an ITF barcode is highly dense, only a relative small area is required to encode a relatively large amount of data.

As used herein, the term "barcode-based code" may refer to a machine-readable code using a barcode scheme, such as ITF, but which may not be made up of bars and spaces. By way of example only, such a barcode-based code may be made up of blocks and spaces, rectangles and spaces, or similar. The barcode-based code may be a linear barcode-based code.

In one example, the, or each, machine-readable code may comprise a two-dimensional matrix, such as a QR code, or an AprilTag, or an ArUco marker. Other suitable two-dimensional matrix codes are known to those skilled in the art.

Optionally, the indicia may be readable if only a portion of the indicia is visible. In other words, the indicia may be configured to be decodable if the indicia is only partly visible. Advantageously, this may allow further improved determination of a location even if only a portion of indicia is visible. One example may be a linear barcode, or linear barcode-based code, which is configured to be readable if a portion of the linear (bar)code is visible.

Indicia or objects which are described as being "visible" may alternatively be referred to as being in a field of view of a sensor or a camera, i.e., being visible may refer to the indicia/object/device or portion thereof being in a line of sight of a sensor or a camera, or one of a plurality of sensors or cameras. Visible objects or indicia or portions thereof may alternatively be referred to as being unoccluded. Objects/indicia that are partly visible may be referred to as partially occluded.

Optionally, the indicia may comprise a basic shape, such as a circle, a triangle, or a square.

The information encoded by the, or each, pattern may comprise a position of the pattern on the device. Advantageously, if the device is partially occluded, i.e. if the device is only partially visible, it may be challenging to determine which portion of the device is unoccluded/visible, which may make it more difficult to precisely determine a location of the device. This may be particularly relevant for determining a location of devices which are symmetrical, and which may be provided in various orientations, because such symmetrical devices may appear similar or the same from different angles and different orientations. By determining a position of the pattern on the device, determination of which portion of the device is unoccluded/visible may be facilitated, thus facilitating precise determination of a location of the device.

The information encoded by the, or each, pattern may further comprise at least one of: a type of the device; an identity of the device; and a geometry of the device.

In some embodiments, the method may further comprise at least one of: recording images using at least one visible light camera and determining the at least one, or at least one further, location characteristic of the device based at least partly on the images; collecting data relating to positioning of a device, for example data from one of a gyroscope, an accelerometer, a stepper motor, a position sensor, or an angle sensor, associated with the device, and determining the at least one, or at least one further, location characteristic of the device based at least partly on the data; and emitting electromagnetic radiation and determining based at least partly on data related to emitted electromagnetic radiation reflected by a device, the at least one, or at least one further, location characteristic of the device, optionally wherein the data related to emitted electromagnetic radiation reflected by the device comprises a duration between emitting electromagnetic radiation and receiving reflected electromagnetic radiation.

These techniques for determining a location of an object, i.e. visible light image recognition, using inertial information, and using depth sensors, all have specific drawbacks as already set out. In particular, when using visible light image recognition, and/or depth sensors (in particular ToF sensors), partially occluded objects may not be precisely located. Thus, while these techniques may allow for 3D maps/point clouds/representations of a 3D environment to be created, if one or more objects are only partially visible to the cameras/sensors, it may not be possible to create a precise map/point cloud/3D representation.

By combining the 3D information obtained by these techniques with the information encoded in the determined pattern, improved determination of a location (in particular an orientation/pose) of a device may be achieved.

More generally, in some embodiments, the method further comprises creating a 3D representation of the healthcare setting. In some examples, the 3D representation may be based on data from depth sensors. In some examples, the 3D representation may be based on computer vision of visible light images. In these embodiments, the 3D representation of the healthcare setting may be modified based on the at least one location characteristic of the device determined in dependence on information encoded by the at least one pattern.

Similarly, while data relating to position of a device related to inertial information from a gyroscope, an accelerometer, a stepper motor, a position sensor, or an angle sensor, may be useful, it can only provide limited information regarding a pose and orientation, and thus, further using the information encoded in the determined pattern may result in improved determination of a location of a device.

In embodiments in which the information includes a position of the pattern on the device, combining this information with the further information obtained by one of the above techniques may allow for a pose/configuration and orientation to be determined more precisely, as it can be determined which portion of the device is unoccluded/visible and/or which portion of the device is occluded.

Optionally, the same sensors may be used for detecting electromagnetic radiation for determining at least one pattern, and for the known technique for determining a location of an object. For example, if ToF sensors are used for creating a point cloud/3D representation, the ToF sensors may be used to detect electromagnetic radiation for determining at least one pattern. Advantageously, this may permit more precise locating of a device without the need for further sensors. As e.g. ToF sensors may already be provided in an operating room, advantageously, no new hardware may be required to carry out the method of the first aspect.

The electromagnetic radiation may comprise at least one of: visible light radiation; and infrared radiation. Preferably, the electromagnetic radiation is infrared radiation, optionally near-infrared radiation. Infrared radiation may be particularly suitable in a healthcare setting, such as an operating room, because infrared radiation is not commonly used in these environments, and thus, there is little background IR radiation which may complicate determination of a pattern.

In some embodiments, the healthcare setting is a surgical suite or an operating room.

In some embodiments, the device is one of: a C-arm, a display, a cart, a surgical table, a stretcher, a hospital bed, a surgical boom, and a light head.

In some embodiments the device is a draped device. That is, the device may be configured to be draped, e.g. during surgery. For example, in an operating room, medical devices such as C-arms are commonly draped during surgical procedures to create a sterile field. In embodiments in which the device is a draped device, the electromagnetic radiation may preferably be infrared radiation. That is because infrared radiation advantageously penetrates well through surgical drapes, and thus facilitates determination of patterns.

In embodiments in which ToF sensors are used, the detected electromagnetic radiation emitted or reflected by a device may preferably be infrared radiation, which may allow for the ToF sensors to be used for detecting the electromagnetic radiation required to determine the at least one pattern.

The method may further comprise creating a collision prevention system based at least partly on the determined location of the device in the healthcare setting.

The method may further comprise modifying a collision prevention system based at least partly on the determined location of the device in the healthcare setting The method may further comprise displaying a model of the device in an augmented reality environment.

The method may further comprise notifying a user of the determined location of the device.

The method may further comprise sending the at least one location characteristic to a computer system for positioning/navigating the device.

The method may be a computer-implemented method. In other words, the method may be a method carried out by a computer.

Optionally, the step of determining at least one pattern of the detected electromagnetic radiation may comprise circle fitting.

In some embodiments, the step of determining at least one pattern of the detected electromagnetic radiation may comprise filtering, in particular Kalman filtering.

In some examples, the method may combine depth data from depth sensors; information encoded in patterns, caused by retroreflective machine-readable code, in IR radiation detected by the depth sensors; and data from an accelerometer associated with the device, to provide a method for precisely determining a location, e.g. a position, a configuration/pose, and an orientation, of a device.

In some embodiments, the at least one location characteristic of the device may comprise a position of the device.

In some embodiments, the at least one location characteristic may comprise at least one of: a midpoint of the device; a normal vector of the device; and an orientation of the device.

According to a second aspect of the present disclosure, there is provided an apparatus for determining a location of a device in a healthcare setting. The apparatus comprises: at least one detector configured to detect electromagnetic radiation emitted or reflected by a device; and a processor configured to: determine at least one pattern of the detected electromagnetic radiation; and, in dependence on information encoded by the at least one pattern, determine at least one location characteristic of the device.

The apparatus of the second aspect, by using information encoded in a pattern of electromagnetic radiation emitted or reflected by a device to determine at least one location characteristic of the device, may enable more precise determination of a 3D location of the object, by helping to identify the unoccluded/visible portion of the object. In particular, the method may significantly improve determination of a 3D geometry of a device which is partially occluded.

The apparatus may be an apparatus for determining a location of a medical device in a healthcare setting. Examples of medical devices include C-arms, surgical tables, and the like.

Optionally, the apparatus further comprises at least one emitter configured to emit electromagnetic radiation. By emitting electromagnetic radiation and detecting electromagnetic radiation emitted or reflected by a device, further processing of the detected electromagnetic radiation may be facilitated, because an intensity and source of electromagnetic radiation emitted by the emitter is known.

In some embodiments, the apparatus comprises at least one depth sensor, the, or each, depth sensor comprising one of the at least one detectors and one of the at least one emitters, and the processor is further configured to: determine, based at least partly on data related to emitted electromagnetic radiation reflected by the device and detected by the or each depth sensor, the at least one, or at least one further, location characteristic of the device.

Advantageously, by using at least one depth sensor(s) to determine a location characteristic, and also using the receiver of the or each depth sensor to sense the pattern, duplication of hardware may be avoided.

Preferably, the apparatus comprises a plurality of depth sensors. A plurality of depth sensors may reduce the likelihood of the device being occluded. A plurality of depth sensors may also allow for a more precise point cloud to be created. In one example, the apparatus comprises two, or three, or four, or seven depth sensors.

In one example in which depth sensors are used, the depth sensors may also be used to detect the electromagnetic radiation comprising the pattern.

Each depth sensor may be a ToF sensor.

Optionally, the apparatus further comprises at least one of: at least one visible light camera, the processor being further configured to: determine the at least one, or at least one further, location characteristic of the device based at least partly on images recorded by the at least one visible light camera; and at least one of: a gyroscope, an accelerometer, a stepper motor, a position sensor, and an angle sensor associated with the device and configured to provide, to the processor, data relating to positioning of the device, the processor being further configured to: determine the at least one, or at least one further, location characteristic of the device based at least partly on the data.

More generally, in some embodiments, the apparatus comprises a means for creating a 3D representation of the healthcare setting. In some examples, the means for creating a 3D representation of the healthcare setting comprises at least one of: a depth sensor such as a ToF sensor, and a visible light camera.

Advantageously, by combining a 3D representation of the healthcare setting, e.g. created using a point cloud based on depth sensors, and the at least one location characteristic of the device determined in dependence on information encoded by the at least one pattern, more precise locating of a device may be achieved, particularly if the device is partly occluded.

In these embodiments, the processor may be configured to modify the 3D representation of the healthcare setting based on the at least one location characteristic of the device determined in dependence on information encoded by the at least one pattern.

Optionally, the means for collecting data to create a 3D representation of the healthcare setting, and the detector, are the same.

The healthcare setting may be a surgical suite or an operating room.

Optionally, the device is one of: a C-arm, a display, and a light head.

In some embodiments, the device is a draped device. That is, the device may be configured to be draped. For example, in an operating room, medical devices such as C-arms are commonly draped during surgical procedures to create a sterile field.

In embodiments in which the device is a draped device, the electromagnetic radiation may preferably be infrared radiation. That is because infrared radiation advantageously penetrates through surgical drapes well, and thus facilitates determination of patterns.

In embodiments in which ToF sensors are used, the detected electromagnetic radiation emitted or reflected by a device may preferably be infrared radiation, which may allow for the ToF sensors to be used for detecting the electromagnetic radiation required to determine the at least one pattern.

The electromagnetic radiation may comprise at least one of: visible light radiation; and infrared radiation. Preferably, the electromagnetic radiation is infrared radiation, optionally near-infrared radiation. Infrared radiation may be particularly suitable in a healthcare setting, such as an operating room, because infrared radiation is not commonly used in these environments, and thus, there is little background IR radiation which may complicate pattern determination.

According to a third aspect of the present disclosure, there is provided a system for determining a location of a device in a healthcare setting. The system comprises: an apparatus according to the second aspect; and at least one radiation modifier, on the device, configured to cause a pattern of electromagnetic radiation to be emitted or reflected by the device.

The at least one electromagnetic radiation modifier may allow for desired information to be encoded in the pattern of electromagnetic radiation.

The system may be a system for determining a location of a medical device in a healthcare setting, and the device may be a medical device.

The, or each, at least one electromagnetic radiation modifier may be painted on, adhered to, or fastened to, the device.

The system may comprise a plurality of electromagnetic radiation modifiers, each electromagnetic radiation modifier configured to cause a pattern of electromagnetic radiation to be emitted or reflected by the device. By providing a plurality of electromagnetic radiation modifiers, a plurality of patterns of electromagnetic radiation may be caused, thus improving versatility, in particular if a partially occluded device is to be located. For example, if a plurality of electromagnetic radiation modifiers is provided, but a device is partially occluded, at least one of the plurality of modifiers may still be determinable because it is on the portion of the device not occluded.

Further optionally, each pattern encodes different information. By encoding different information in at least two patterns, further improved locating may be achieved, because one can identify, if only one pattern is visible, which pattern is visible, and by using any further encoded information, improved accuracy in locating the device may be achieved.

In some embodiments, the or each at least one radiation modifier comprises at least one of: a passive radiation modifier configured to affect a reflection of electromagnetic radiation; and an active source of electromagnetic radiation configured to emit electromagnetic radiation.

Advantageously, a passive electromagnetic radiation modifier may be simpler, because it does not require, e.g. electrical, activation. Advantageously, an active electromagnetic radiation source does not rely on external electromagnetic radiation, thus it allows the modifier to be independent of external factors.

In some examples, the, or each, passive electromagnetic radiation modifier comprises at least one of: a reflector configured to reflect a greater proportion of incident electromagnetic radiation than the device, and an absorber configured to absorb a greater proportion of incident electromagnetic radiation than the device. Advantageously, a reflector may allow for relatively low intensity radiation to be detected, as the reflector reflects a greater proportion of incident electromagnetic radiation. On the other hand, an absorber may be suitable only if incident electromagnetic radiation is sufficiently high, so that the radiation reflected by the device is sufficiently high to be distinguished from the (smaller proportion) reflected by the absorber. However, an absorber may be particularly suitable if the device reflects a relatively greater proportion of incident electromagnetic radiation.

The, or each, reflector may preferably be a retroreflector. A retroreflector may facilitate determination of a pattern, as it reflects radiation with minimal scattering. As such, a retroreflector may provide a maximum contrast.

In some examples, the, or each, active electromagnetic radiation source is configured to emit electromagnetic radiation continuously, or intermittently. Advantageously, electromagnetic radiation being emitted continuously may facilitate determination of a pattern. On the other hand, intermittently emitting electromagnetic radiation may allow for information to be encoded in the intermittent signal.

The, or each, active electromagnetic radiation source may comprise at least one light-emitting diode (LED), in particular an infrared (IR) LED.

In some examples, the, or each, passive electromagnetic radiation comprises at least one geometric feature on a surface of the device, the geometric feature comprising at least one of: an indentation, and a projection.

The, or each, electromagnetic radiation modifier may be arranged to form indicia in the pattern. By arranging the electromagnetic radiation modifier to form indicia in the pattern, information may be easily encoded in the pattern. Greater information density may also be achieved.

In some examples, the indicia comprise at least one machine-readable code, such as at least one barcode or at least one barcode-based code. Advantageously, a barcode, or a barcode-based code, may allow for a relatively large amount of information to be encoded in a simple manner.

Optionally, the, or each, barcode comprises a linear barcode. Advantageously, a linear barcode may be easy to read and may be easy to provide to encode information. One suitable example is an Interleaved 2 of 5 (ITF) barcode. ITF is a relatively high density (i.e. space-efficient) numeric-only barcode. Advantageously, because an ITF barcode is highly dense, only a relative small area is required to encode a relatively large amount of data.

As used herein, the term "barcode-based code" may refer to a machine-readable code using a barcode scheme, such as ITF, but which is not made up of bars and spaces. By way of example only, such a barcode-based code may be made up of blocks and spaces, rectangles and spaces, or similar. The barcode-based code may be a linear barcode-based code.

In one example, the, or each, machine-readable code may comprise a two-dimensional matrix, such as a QR code, or an AprilTag, or an ArUco marker. Other suitable two-dimensional matrix codes are known to those skilled in the art.

Optionally, the indicia may be configured to be readable if only a portion of the indicia is visible. In other words, the indicia may be configured to be decodable if the indicia is only partly visible. Advantageously, this may allow further improved determination of a location even if only a portion of the device, and a portion of the indicia, is not occluded. One example may be a linear barcode, or linear barcode-based code, which is configured to be readable if a portion of the (bar)code is unoccluded.

In some embodiments, the information encoded by the, or each, pattern comprises a position of the pattern on the device. Advantageously, if the device is only partially visible, it may be challenging to determine which portion of the device is visible, which may complicate precise determination of a location of the device. This may be particularly relevant for determination of a location of a device which is symmetrical because a symmetrical device may appear the same from different angles and different orientations. By determining a position of the pattern on the device, determination of the visible portion of the device may be facilitated, thus facilitating precise determination of a location of the device.

The information encoded by the, or each, pattern may further comprise at least one of: a type of the device; an identity of the device; and a geometry of the device.

Optionally, the, or each, at least one electromagnetic radiation modifier may be removable. Further optionally, the, or each, at least one electromagnetic radiation modifier may be reusable.

Further features of the second and third aspects of the present disclosure are described above in relation to the first aspect of the present disclosure.

According to a further aspect, there is provided a computer-readable storage medium comprising instructions which, when executed by a computer, cause the computer to carry out the method according to the first aspect.

Where functional components are referred to in apparatus embodiments for carrying out various steps of the described method(s) it will be understood that these components may be implemented in hardware, in software, or a combination of the two. When implemented in hardware, the components may be implemented as one or more hardware components, such as one or more application specific integrated circuits. When implemented in software, the components may be implemented as one or more computer programs that are executed on one or more processors.

It will be appreciated that features described in relation to one aspect of the present disclosure may also be applied equally to all of the other aspects of the present disclosure. Features described in relation to the first aspect of the present disclosure may be applied equally to the second and third aspects of the present disclosure and vice versa. For example, method features described in relation to the first aspect may be applied, mutatis mutandis, to the apparatus and system of the second and third aspects, or to the computer-readable storage medium of the further aspect.

### BRIEF DESCRIPTION OF DRAWINGS

The disclosure will be further described, by way of example only, with reference to the accompanying drawings, in which:
Figure 1 shows a schematic illustration of a system according to the present disclosure;
Figure 2a shows a block diagram of a system according to the present disclosure;
Figure 2b shows a photograph of a part of a system according to the present disclosure;
Figure 3 shows an infrared image of machine-readable codes on a C-arm;
Figure 4 shows an inverted version of the infrared image of Figure 3;
Figure 5 shows a C-arm, detected using depth sensors and machine-readable code, with its midpoint and normal vector; and
Figures 6 to 10 show flow diagrams illustrating steps of example methods according to the present disclosure.

### DETAILED DESCRIPTION OF DRAWINGS

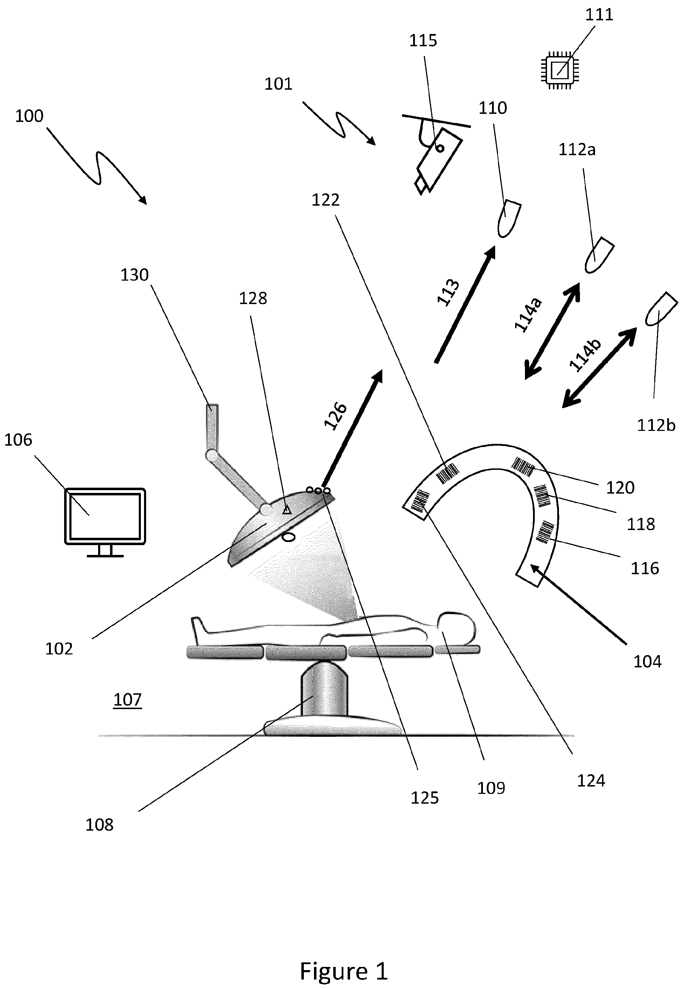

Figure 1 shows a schematic illustration of a system 100 for determining a location of a device, such as a surgical light head 102, a C-arm 104, or a display 106, in a healthcare setting, such as an operating room 107. An adjustable patient support apparatus, such as a surgical table 108, is provided in the operating room 107, to support a patient 109.

The system 100 comprises an apparatus 101 for determining a location of a device 102, 104, 106. The apparatus 101 comprises a detector 110, configured to detect infrared (IR) radiation 113 emitted or reflected by a device.

The apparatus 101 includes a processor 111, operatively coupled to the detector 110, configured to determine at least one pattern of the detected IR radiation 113; and, in dependence on information encoded by the at least one pattern, i.e. information encoded in the at least one pattern, determine at least one location characteristic of the device.

The apparatus 101 comprises at least one Time-of-Flight (ToF) IR depth sensor 112a, 112b, configured to emit IR radiation 114a, 114b, and receive reflected IR radiation 114a, 114b from the operating room 107, including IR radiation reflected by a device. The reflected IR radiation received by the ToF IR depth sensors 112a, 112b may be used to compute a point cloud, creating a 3D representation of operating room 107, including devices 102, 104, 106.

The processor 111 is operatively coupled to the ToF IR depth sensors 112a, 112b. The processor 111 is configured to determine, based at least partly on data related to emitted IR radiation reflected by the device and detected by the depth sensor 112a, 112b, at least one, or at least one further, location characteristic of the device 102, 104, 106.

The apparatus 101 may also include at least one visible light camera 115, configured to capture visible light images of the operating room 107, and operatively coupled to the processor 111. The processor 111 is configured to determine at least one, or at least one further, location characteristic of the device 102, 104, 106 based at least partly on images recorded by the at least one visible light camera 115.

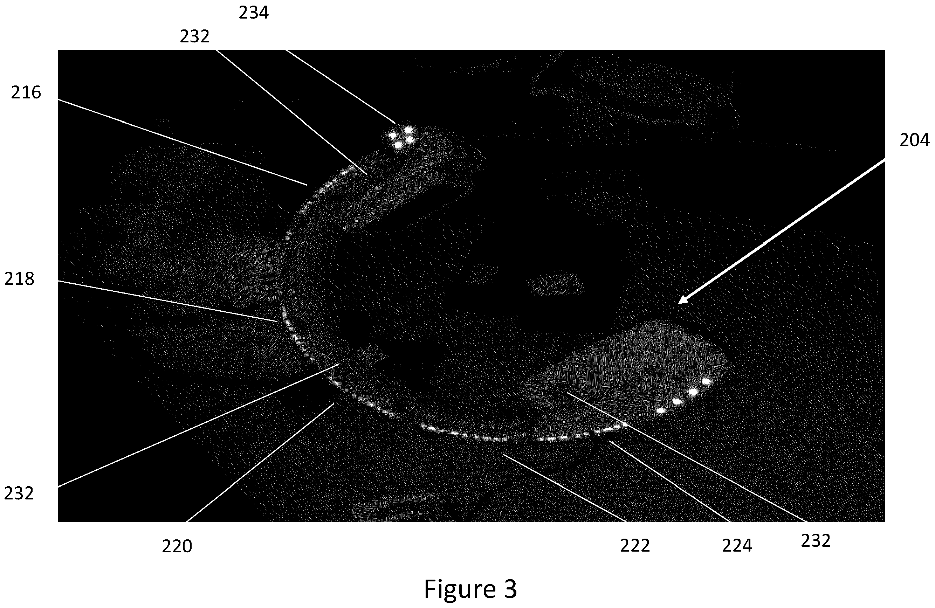

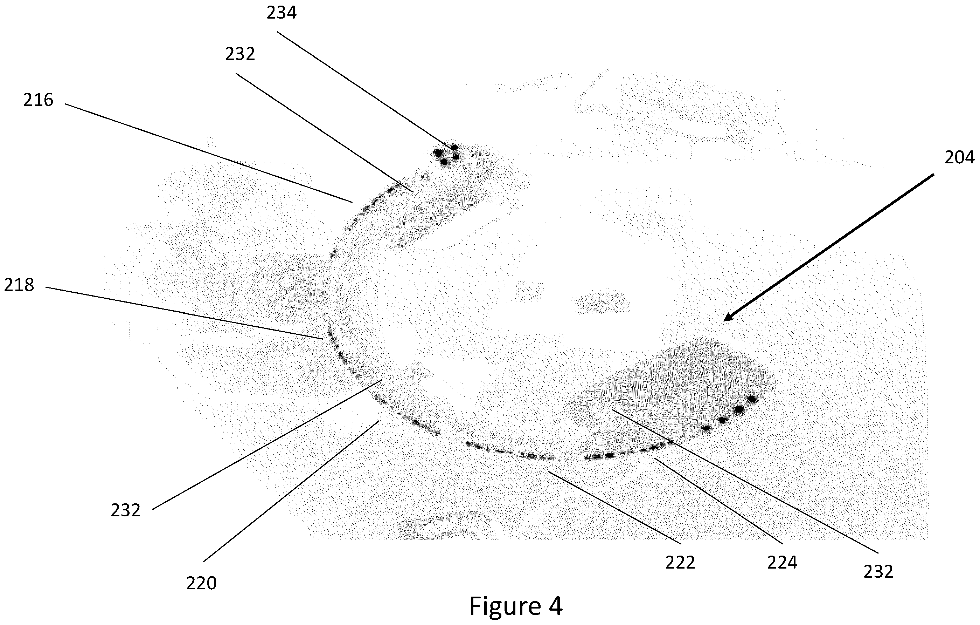

As part of the system 100, a plurality of retroreflective machine-readable codes 116, 118, 120, 122, 124 is provided on one of the devices, in this example, on a surface of the C-arm 104. Because the retroreflective machine-readable codes 116, 118, 120, 122, 124 reflect a greater proportion of incident IR radiation 114a, 114b emitted by the ToF IR depth sensors 112a, 112b, a greater proportion of reflected IR radiation 114a, 114b is received by the ToF IR depth sensors 112a, 112b. Thus, the barcodes 116, 118, 120, 122, 124 are visible in a 3D representation of the operating room 107 as areas of greater intensity of IR radiation, as explained below with reference to Figures 3 and 4.

Each machine-readable code 116, 118, 120, 122, 124 is configured to produce a pattern in the detected electromagnetic radiation which encodes information related to a location characteristic of the C-arm 104. Each machine-readable code 116, 118, 120, 122, 124 encodes different information, in particular, each machine-readable code 116, 118, 120, 122, 124 encodes at least a position of the respective barcode 116, 118, 120, 122, 124 on the C-arm 104. Thus, even if only a portion of the C-arm 104 is visible to the ToF IR depth sensors 112a, 112b, i.e. even if a portion of the C-arm 104 is occluded by another object or a person, at least one of the barcodes 116, 118, 120, 122, 124 may be visible to the sensors 112a, 112b.

By decoding the machine-readable code(s) which is/are not occluded, it may be determined which portion of the C-arm 104 is in view of the depth sensors 112a, 112b. The position of each detected, unoccluded code on the C-arm 104 may also be precisely determined.

Each machine-readable code 116, 118, 120, 122, 124 is a linear ITF barcode.

As part of the system 100, a plurality of IR LEDs 125 may be provided on one of the devices, in this example, on a surface of the surgical light 102. The plurality of IR LEDs 125 is configured to emit IR radiation 126, which may be detected by the detector 110 and/or the depth sensors 112a, 112b. The IR LEDs 125 are arranged to create a pattern in the detected IR radiation which encodes a position of the pattern on the surgical light 102.

As part of the system 100, an accelerometer, an angle sensor or a gyroscope may be provided on at least one of the devices. In this example, an accelerometer 128 is provided on the surgical light 102 carried by positioning arm 130. The accelerometer 128 is operatively coupled to the processor 111, and provides data relating to positioning of the surgical light 102. The processor 111 is configured to determine the at least one, or at least one further, location characteristic of the surgical light based at least partly on the data.

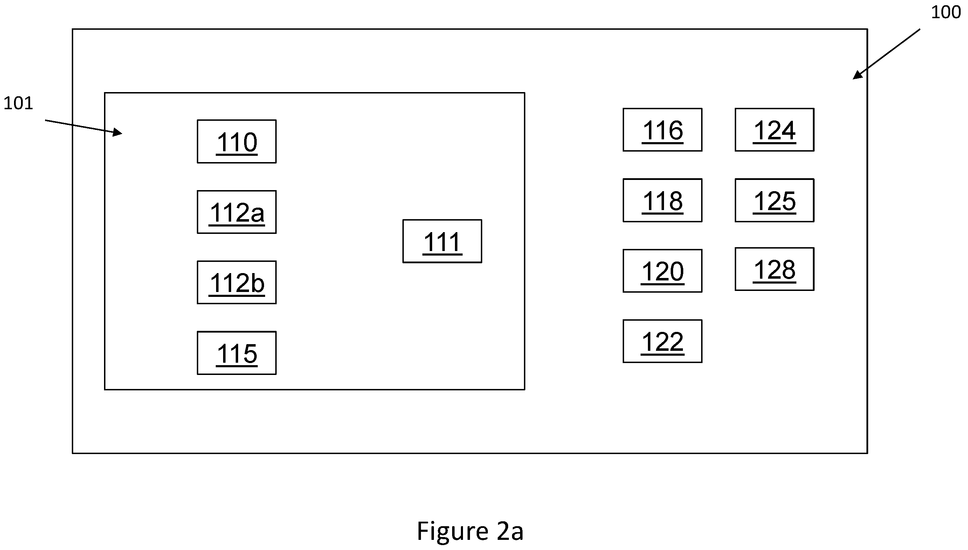

Figure 2a shows the various components of the system 100, and the apparatus 101 which the system 100 comprises, and their relationship.

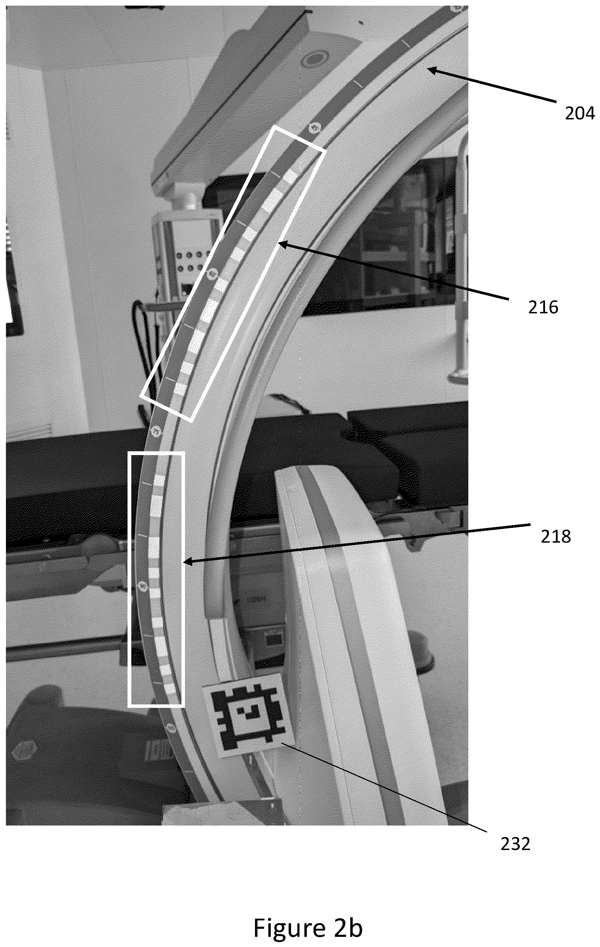

Figure 2b shows a portion of an example of a device, specifically a C-arm 204, to which retroreflective machine-readable codes 216, 218 have been retrofitted by adhering retroreflective markers. In this manner, the retroreflective machine-readable codes 216, 218 create desired patterns in the infrared radiation detected by the depth sensor(s) 112a, 112b, by causing areas of increased intensity, as shown in Figure 3 and discussed below. In this example, the retroreflective machine-readable codes 216, 218 are linear barcode-based readable codes, specifically ITF-based readable codes, made up of blocks and spaces.

A two-dimensional matrix code 232, an ArUco marker, is further attached to the C-arm 204. In this example, the two-dimensional code 232 is configured to be visible to a visible light camera. However, in other examples, a two-dimension matrix code 232 which is retroreflective to IR radiation, or comprises active sources of IR radiation, may be provided.

As shown in Figure 3, which is a two-dimensional representation of detected IR radiation reflected by C-arm 204, the retroreflective machine-readable codes 216, 218, 220, 222, 224 appear as bright spots in the monochrome image. As also shown in Figure 3, the C-arm 204 itself also reflects IR radiation emitted by the depth sensors 112a, 112b, thus allowing for a location of the C-arm 204 to be determined based on ToF measurements. Other objects in the background also reflect IR radiation, as best seen in the inverted monochrome image of Figure 4.

The ArUco markers 232 attached to the C-arm 204 are barely visible in the IR image of Figure 3 - however, in other examples, as noted above, the ArUco markers 232 may be retroreflective to IR radiation, or comprise active IR radiation sources.

A retroreflective two-dimensional symbol 234 is shown in the IR image, which may encode a position near a first end of the C-arm 204. The inverted image of Figure 4 allows some of the background features to be seen more easily.

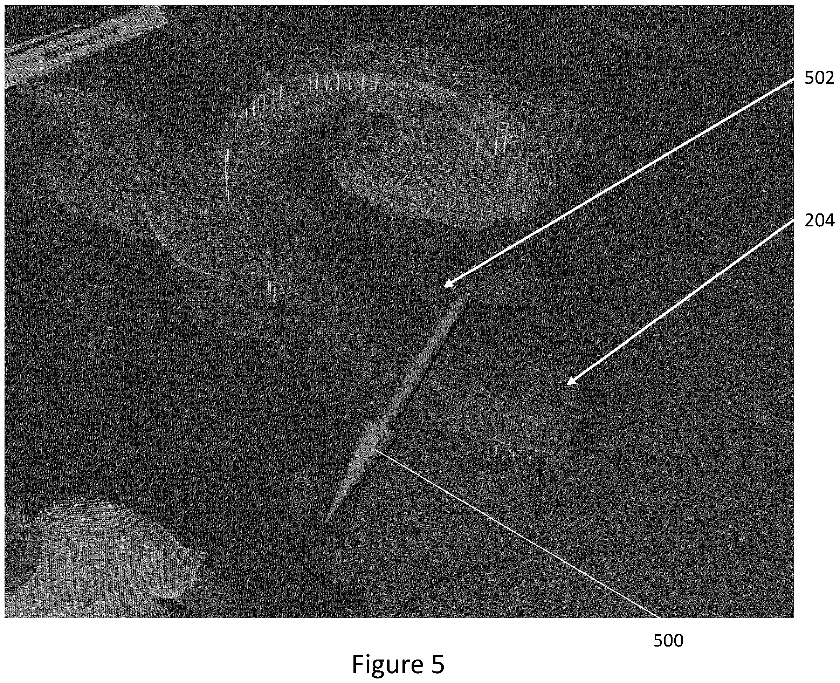

Figure 5 shows the C-arm 204, as detected using depth sensors 212a, 212b, with its midpoint 502 and normal vector 504 as determined by the processor 111, based on depth data and information encoded by the machine-readable codes. The patterns created by some of the machine-readable codes are visible in Figure 5, whereas some of the machine-readable codes are occluded because of the orientation of the C-arm, and thus, the patterns created by those occluded machine-readable codes are not visible in Figure 5.

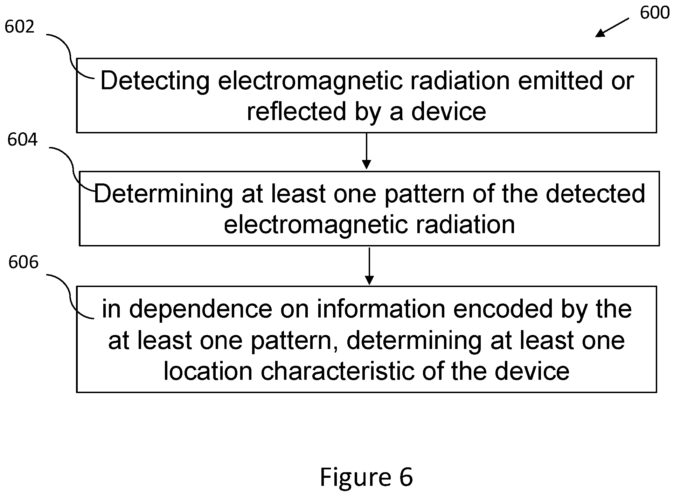

Figure 6 shows a flowchart representing a method 600 according to the present disclosure. The method 600 comprises a step of detecting 602 electromagnetic radiation emitted or reflected by a device. In one example, the electromagnetic radiation being detected is IR radiation.

The method 600 further comprises a step of determining 604 at least one pattern of the detected electromagnetic radiation. In one example, the pattern is caused by a retroreflective machine-readable code, e.g. a linear barcode-based code, as shown, e.g., in Figure 5.

The method 606 further comprises a step of, in dependence on information encoded by the at least one pattern, determining 606 at least one location characteristic of the device. In one example, the information encoded by the pattern includes a position of the pattern on the device, i.e. the position of a code causing the pattern on the device. In one example, in addition to the position of the pattern/code, the information comprises a type and a dimension of the device. In one example, the at least one location characteristic of the device may comprise a position of the device. In one example, the at least one location characteristic may comprise at least one of a midpoint of the device and a normal vector of the device.

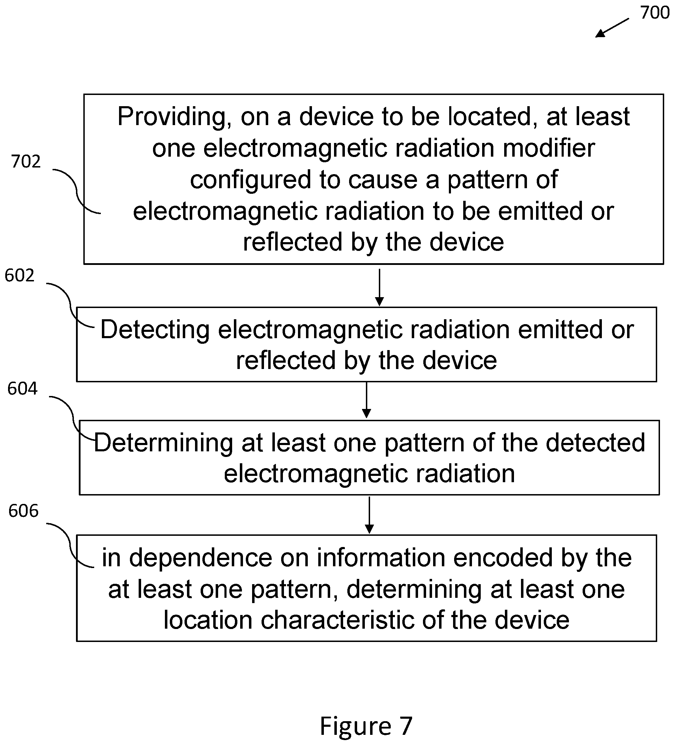

Figure 7 shows a flowchart representing a further method 700 according to the present disclosure. The method 700 comprises the steps 602, 604, and 606 of method 600. The method 700 further comprises providing 702, on a target device, at least one electromagnetic radiation modifier configured to cause a pattern of electromagnetic radiation to be emitted or reflected by the device. In one example, the at least one electromagnetic radiation modifier may be provided by adhering to the device, or by fastening to the device, or by painting, e.g. by spraying, onto the device.

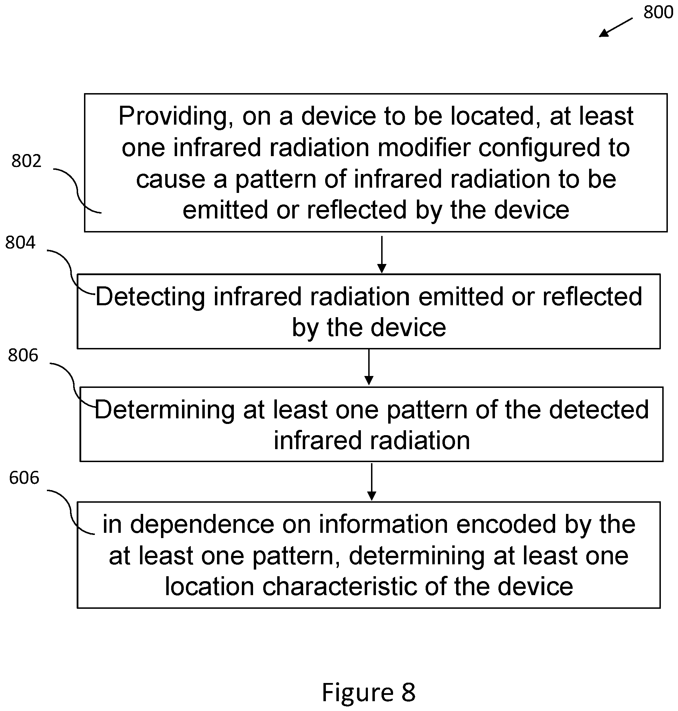

Figure 8 shows a flowchart representing a more specific method 800 of the example of method 700. In particular, method 800 comprises a step of providing 802, on a target device, at least one infrared radiation modifier configured to cause a pattern of infrared radiation to be emitted or reflected by the device. The method 800 further comprises a step of detecting 804 infrared radiation emitted or reflected by the device and a step of determining 806 at least one pattern of the detected infrared radiation. The method 800 further comprises a step of, in dependence on information encoded by the at least one pattern, determining 608 at least one location characteristic of the device.

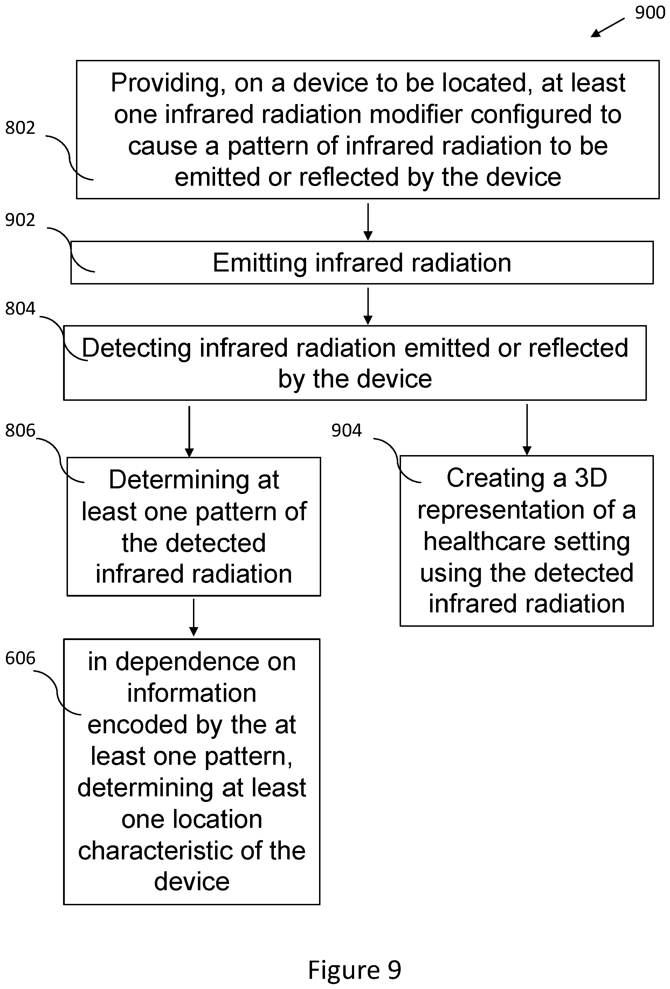

Figure 9 shows a flowchart representing a further example method 900, based on method 800. Method 900 comprises steps 802, 804, 806, and 606 of method 800. The method 900 further comprises a step of emitting 902 infrared radiation. The method further comprises a step of creating 904 a 3D representation of a healthcare setting (in which the device is located) using the detected infrared radiation.

As method 900 comprises steps 806 and 606, as well as step 904, the at least one location characteristic determined in dependence on information encoded by the at least one pattern may be combined with the 3D representation, which may be based on e.g. a point cloud of depth data, to more precisely determine a location, i.e. an orientation, a position, a pose, a configuration etc. of the device.

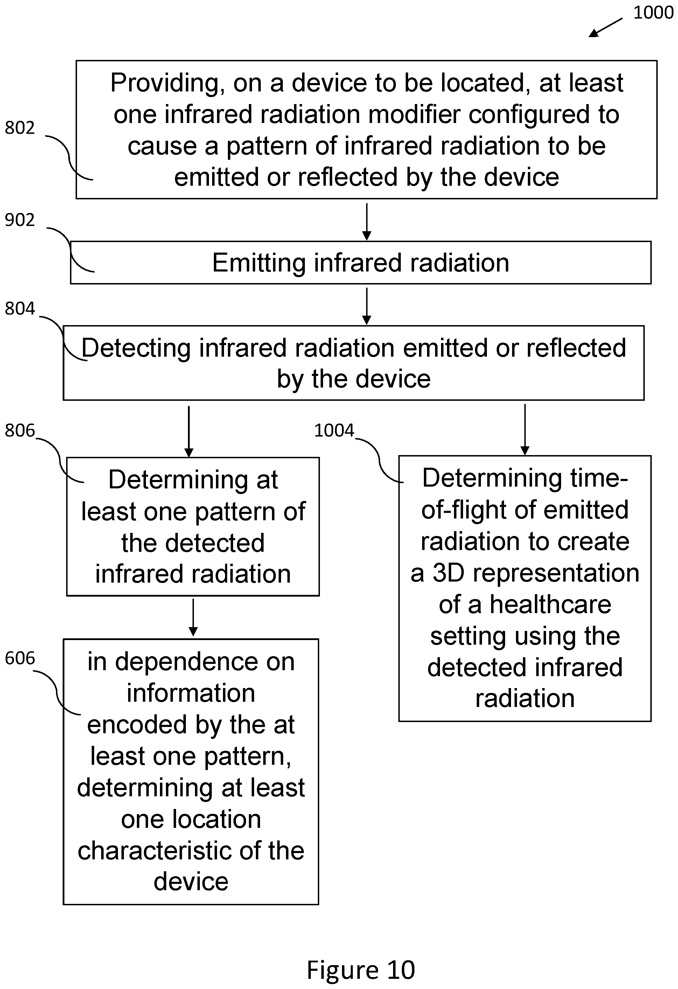

Method 1000, represented by the flowchart of Figure 10, is based on method 900. Method 1000 comprises steps 802, 902, 804, 806, and 606. Method 1000 further comprises, instead of step 904, the more specific step of determining 1004 time-of-flight of emitted radiation to create a 3D representation of a healthcare setting using the detected infrared radiation. For example, the 3D representation may be created based on depth data captured by ToF depth sensors, which emit infrared radiation and create a 3D representation based on a time of flight of emitted infrared radiation reflected by the device, as set out above.

It will be appreciated that the above described embodiments are exemplary embodiments of the disclosure only. It will also be appreciated that features described above in relation to one embodiment of the disclosure may also be applied to other embodiments of the disclosure.

## Claims

1. A method of determining a location of a device in a healthcare setting, the method comprising:
detecting electromagnetic radiation emitted or reflected by a device to be located;
determining at least one pattern of the detected electromagnetic radiation;
and, in dependence on information encoded by the at least one pattern,
determining at least one location characteristic of the device.

2. The method according to claim 1, further comprising providing, on a device to be located, at least one electromagnetic radiation modifier configured to cause a pattern of electromagnetic radiation to be emitted or reflected by the device.

3. The method according to claim 2, wherein a plurality of electromagnetic radiation modifiers are provided, each electromagnetic radiation modifier configured to cause a pattern of electromagnetic radiation to be emitted or reflected by the device, and optionally wherein each pattern encodes different information.

4. The method according to claim 2 or 3, wherein the, or each, electromagnetic radiation modifier comprises at least one of:
a passive electromagnetic radiation modifier, optionally wherein the passive electromagnetic radiation modifier comprises at least one of:
a reflector configured to reflect a greater proportion of incident electromagnetic radiation than the device, wherein the reflector is preferably a retroreflector, and
an absorber configured to absorb a greater proportion of incident electromagnetic radiation than the device; and
an active electromagnetic radiation source configured to emit electromagnetic radiation,
optionally wherein the or each active electromagnetic radiation source is configured to emit electromagnetic radiation continuously, or intermittently.

5. The method according to claim 2, 3, or 4, wherein the, or each, electromagnetic radiation modifier is arranged to form indicia, the indicia preferably comprising at least one machine-readable code, such as a barcode or a barcode-based code, in the pattern.

6. The method according to any preceding claim, wherein information encoded by the or each pattern comprises a position of the pattern on the device.

7. The method according to any preceding claim, further comprising at least one of:
recording images using at least one visible light camera and determining the at least one, or at least one further, location characteristic of the device based at least partly on the images;
collecting data relating to positioning of a device, for example data from one of a gyroscope, an accelerometer, or a stepper motor associated with the device, and determining the at least one, or at least one further, location characteristic of the device based at least partly on the data; and
emitting electromagnetic radiation and determining based at least partly on data related to emitted electromagnetic radiation reflected by a device, the at least one, or at least one further, location characteristic of the device,
optionally wherein the data related to emitted electromagnetic radiation reflected by the device comprises a duration between emitting electromagnetic radiation and receiving reflected electromagnetic radiation.

8. The method according to any preceding claim, wherein the electromagnetic radiation comprises at least one of: visible light radiation; and, infrared radiation, preferably wherein the electromagnetic radiation is infrared radiation, optionally near-infrared radiation.

9. The method according to any preceding claim, wherein the at least one pattern comprises a plurality of patterns, and optionally wherein each pattern encodes different information.

10. An apparatus for determining a location of a device in a healthcare setting, the apparatus comprising:
at least one detector configured to detect electromagnetic radiation emitted or reflected by a device to be located; and
a processor configured to:
determine at least one pattern of the detected electromagnetic radiation; and, in dependence on information encoded by the at least one pattern, determine at least one location characteristic of the device.

11. The apparatus according to claim 10, further comprising at least one emitter configured to emit electromagnetic radiation, optionally wherein the apparatus comprises at least one depth sensor, the, or each, depth sensor comprising one of the at least one detectors and one of the at least one emitters, and wherein the processor is further configured to: determine, based at least partly on data related to emitted electromagnetic radiation reflected by the device and detected by the or each depth sensor, the at least one, or at least one further, location characteristic of the device.

12. The apparatus according to claim 10 or 11, wherein the apparatus further comprises at least one of:
at least one visible light camera, the processor being further configured to:
determine the at least one, or at least one further, location characteristic of the device based at least partly on images recorded by the at least one visible light camera; and
at least one of: a gyroscope, an accelerometer, and a stepper motor associated with the device and configured to provide, to the processor, data relating to positioning of the device, the processor being further configured to: determine the at least one, or at least one further, location characteristic of the device based at least partly on the data.

13. A system for determining a location of a device in a healthcare setting, the system comprising:
an apparatus according to claim 10, 11, or 12; and
at least one radiation modifier, on the device to be located, configured to cause a pattern of electromagnetic radiation to be emitted or reflected by the device.

14. The system according to claim 13, wherein the or each at least one radiation modifier comprises at least one of:
a passive radiation modifier configured to affect a reflection of electromagnetic radiation; and
an active source of electromagnetic radiation configured to emit electromagnetic radiation.

15. The system according to claim 13 or 14, wherein the, or each, electromagnetic radiation modifier is arranged to form indicia, the indicia preferably comprising at least one machine-readable code, such as a barcode or a barcode-based code.
